# EUROPEAN PATENT APPLICATION

(11) **EP 3 320 905 A1**
(43) Date of publication of application: **16.05.2018**
(21) Application number: 16823926.7
(22) Date of filing: 08.07.2016
(51) Int. Cl.: A61K 31/6615, A61K 31/4415, A61P 3/10

(54) **USE OF PHYTIC ACID OR THE SALTS THEREOF, ALONE OR COMBINED WITH B6 VITAMERS, FOR PREVENTING THE FORMATION OF ADVANCED GLYCATION END-PRODUCTS**

(30) Priority: 10.07.2015 ES 201531010
(71) Applicant: Universitat de les Illes Balears, 07120 Palma de Mallorca (Islas Baleares) (ES); Servei de Salut de Les Illes Balears - Ibsalut, 07120 Palma de Mallorca (Islas Baleares) (ES)
(72) Inventor: GRASES FREIXEDAS, Félix, 07120 Palma de Mallorca (Islas Baleares) (ES); COSTA BAUZA, Antonia, 07120 Palma de Mallorca (Islas Baleares) (ES); ADROVER ESTELRICH, Miquel, 07120 Palma de Mallorca (Islas Baleares) (ES); BERGA MONTANER, Francisco, 07120 Palma de Mallorca (Islas Baleares) (ES); SANCHÍS CORTÉS, Pilar, 07120 Palma de Mallorca (Islas Baleares) (ES); MASMIQUEL COMAS, Luis, 07120 Palma de Mallorca (Islas Baleares) (ES); RIVERA IROGOIN, Rosmeri, 07120 Palma de Mallorca (Islas Baleares) (ES); FORTUNY MARQUÉS, Regina, 07120 Palma de Mallorca (Islas Baleares) (ES)
(74) Representative: ZBM Patents ApS
(86) International application number: PCT/ES2016/070515
(87) International publication number: WO 2017/009506

(57) **Abstract**

The invention relates to the use of phytate or any of the salts thereof for preventing the formation of advanced glycation end-products (AGE), toxic products that are produced in pathologies such as diabetes, either alone or combined with B6 vitamins such as pyridoxamine and forming part of a medicament or a pharmaceutical composition produced so as to be administered via oral, rectal, subcutaneous, intra-arterial, intramuscular, intraspinal, intracranial, intravenous or inhalation routes.

## Description

The present invention relates to the use of phytate or any of the salts thereof to prevent the formation of advanced glycation end-products (EGE) that are produced in pathologies such as diabetes, whether alone or in combination with B6 vitamers such as pyridoxamine and forming part of a medicament or pharmaceutical composition prepared so as to be administered via oral, rectal, subcutaneous, intra-arterial, intramuscular, interspinal, intracranial, intravenous, or inhalation routes. Both phytate and B6 vitamers exhibit the ability to inhibit the formation of AGEs, for which reason they can be used for therapeutic purposes in the treatment or prevention of diseases that appear as a consequence of the formation of AGEs, such as neuropathy, nephropathy, atherosclerosis, vascular calcification, and neurodegenerative diseases (Alzheimer's and Parkinson's disease).

### Prior Art

Diabetes mellitus (DM) is a chronic pathology that leads to the development of various metabolic disorders due to an increase in the concentration of glucose in the blood. The increase in glycemic levels in diabetic patients can lead to the development of a wide range of metabolic disorders, the specific seriousness of which will depend on the particular physiopathology of each diabetic. Standing out among these are nephropathy or diabetic cardiopathy, as well as the high predisposition on the part of diabetics to experience various neurodegenerative events. These pathologies are directly induced by hyperglycemia as a result of the non-specific chemical interaction between reducing sugars (essentially glucose) and different biological macromolecules. This process, known by the name of non-enzymatic glycation, results in the structural and functional deterioration of the molecules on which it occurs, thus leading to the pathological development.

Although glycation can occur in the majority of biological molecules, such as lipid bilayers or on DNA, most of the pathologies associated with DM appear due to the non-enzymatic glycation of proteins (GP) that have a low turnover rate. Protein glycation thus begins with condensation among the proteinaceous amine groups (present in the side chains of the arginines and lysines) with glucose or other reducing carbohydrates. The product of this condensation forms a carbohydrate-based Schiff base, which rearranges irreversibly to yield an Amadori compound. This compound then evolves by means of various mechanisms until the final products of glycation are formed, which are commonly referred to as AGEs ("advanced gycation end-products"). In parallel, the glucose, the Schiff base, and the Amadori compound can undergo auto-oxidation reactions, which produce free radicals and highly reactive carbonyl compounds, which can react again with other amino acids, inducing the formation of new AGEs and furthering the protein damage that was initiated by the glucose in the first place.

The AGEs thus constitute a heterogeneous family of compounds whose chemical nature depends on the type of protein residue that is reacting, the environment, and the type of glycating agent. Even though its pathological action is centered in the structural and functional modification that its formation entails (e.g., the glycated hemoglobin HbA1c has a lower affinity to oxygen than the native one), its interaction with its specific receptors (known as RAGEs) does not induce only a pro-inflammatory process, but also increases the oxidative stress, which has an essential impact on the vascular system. In addition, it has also been observed that AGEs induce cellular apoptosis.

All in all, the levels of AGEs circulating in the blood of patients with DM are generally much higher than in healthy people. Indeed, those levels are the result of a delicate balance between the endogenous production, exogenous ingestion, endogenous degradation thereof (by means of specific enzymes such as glyoxalases I, II, and carbonyl reductase) and the renal excretion thereof. The alteration of this balance by means of any pathological factor (such as hyperglycemia) can lead to a pathological development.

A large part of the therapeutic strategy aimed at diminishing the predisposition of diabetics to suffer from pathologies induced by an increase in circulating AGEs has been focused on the search for small compounds to inhibit their formation. One of the most-used compounds at the clinical level is metformin, which reduces the production of glucose in the liver and increases the muscular absorption thereof. It has also been seen to be capable of substantially reducing the formation of AGEs both *in vitro* and *in vivo* by scavenging carbonyl compounds with a high glycating capacity that are formed during the oxidation of glucose, Schiff base, and Amadori compound (e.g., glyoxal or methylglyoxal). However, this is the only mechanism of action by means of which metformin can inhibit glycation, since it is not capable of scavenging the free radicals formed during protein glycation or chelating the Cu²⁺ or Fe³⁺ ions that catalyze the different stages of protein glycation. This means that it is only able to act effectively at high doses (500-800 mg every 8-12 hours), which can cause side effects in the medium and long term in one of every ten patients treated, such as nausea, vomiting, diarrhea, or loss of appetite. Because of this, a large part of the current research in the field of DM is focused on the search for natural molecules that are able to inhibit the formation of AGEs through different complementary mechanisms of action. This would enable the administration of lower doses, thereby minimizing the adverse effects.

Phytate is a naturally occurring phosphorus compound that has been administered in diabetic patients with the purpose of controlling blood sugar levels (EP 0342955) or reducing the symptoms associated with diabetes, such as circulatory problems, paresthesia, or pain (Sanchez Lopez E. Rev. Clin. Esp. 15 Nov 1969, vol. 115, no. 3, pages 219-226).

Another natural compound of interest is pyridoxamine (PM), a vitamer of vitamin B6 that is found abundantly in the liver. When administered, it reduces the formation of AGEs considerably both in animal models and in diabetic individuals (application WO 2004112788) by means of different mechanisms that act in a complementary manner: a) scavenging of carbonyl compounds with a high glycation capacity; b) formation of weak-natured metallic complexes with Cu²⁺y and Fe³⁺ ions, which catalyze the protein glycation; c) neutralization of the free radicals formed during protein glycation; and d) cellular apoptosis. The discovery of these characteristics of a high pharmacological potential has led to its protection as treatment for combatting diabetic retinopathy (WO 2004112788), as part of a topical composition for reducing the formation of AGEs on the skin (US 7,666,442 82), or as an inhibitor of the complications associated with diabetes (US 2005/0014799 A1). It has also been observed to have a beneficial effect in the treatment of diabetic retinopathy (Stitt A, et al., Diabetes, 2002, 51, 2826-2832).

### Description of the Invention

The object of the present invention is the use of phytate or any of the salts thereof alone or in combination with B6 vitamers to prevent the formation of AGEs and the development of the pathologies associated with diabetes in patients suffering from that disorder, with it being possible for the phytate alone or in combination with the B6 vitamers to form part of a pharmaceutical medicament or composition prepared so as to be administered via oral, rectal, subcutaneous, intra-arterial, intramuscular, interspinal, intracranial, intravenous, or inhalation routes. These AGE formation inhibitors, which are related to the aspects described in the section on the prior art and in the recent discoveries of the applicants regarding the combination of phytic acid and/or pharmaceutically acceptable phytates thereof with B6 vitamers such as pyridoxamine, can be used for therapeutic purposes in the treatment of diseases that appear as a consequence of the formation of AGEs, as is the case with many of the pathologies associated with diabetes. The isolated or combined use of phytic acid and/or phytates thereof with B6 vitamers and/or analogous molecules has not been described before and may prove to be very beneficial in the treatment of diabetic patients, since it can substantially reduce the formation of AGEs.

Therefore, in a first aspect, the present invention relates to the use of a composition that comprises phytate or any of the salts thereof for the manufacture of a medicament for preventing the formation of glycation end-products, or AGEs, in diabetic patients, with the medicament being prepared so as to be administered via oral, rectal, subcutaneous, intra-arterial, intramuscular, interspinal, intracranial, intravenous, or inhalation routes.

In a preferred embodiment, the composition further comprises a B6 vitamer or any of the salts thereof. In an embodiment that is more preferred, the B6 vitamer is selected from among pyridoxine, pyridoxal, or pyridoxamine, most preferably pyridoxamine. In another preferred embodiment, the phytic acid salts are selected from among sodium phytate, potassium phytate, calcium phytate, magnesium phytate, zinc phytate, or combinations thereof. In an embodiment that is more preferred, the phytic acid salt is calcium-magnesium phytate.

In another preferred embodiment, the phytic acid or salts thereof originate from a plant species that is rich in phytic acid or salts thereof, or from a vegetable extract from that species.

Another aspect of the invention relates to the use of a composition which comprises phytic acid or any of the pharmaceutically acceptable salts thereof, combined sequentially, simultaneously, or separately with a B6 vitamer, for the manufacture of a medicament for preventing the formation of AGEs in diabetic patients.

Another aspect of the invention relates to a composition which comprises phytic acid or any of the salts thereof and at least one derivative of vitamin B6 or any of the salts thereof.

In a preferred embodiment, this composition further comprises a B6 vitamer or any of the salts thereof. In a preferred embodiment, the B6 vitamer is selected from among pyridoxine, pyridoxal, or pyridoxamine, most preferably pyridoxamine.

In another preferred embodiment, the phytic acid salt in this composition is selected from among sodium phytate, potassium phytate, calcium phytate, magnesium phytate, zinc phytate, or combinations thereof. More preferably, the phytic acid salt is calcium-magnesium phytate.

In another preferred embodiment, the phytic acid or salts thereof originate from a plant species that is rich in phytic acid or salts thereof, or from a vegetable extract from that species.

In the present invention, the term "phytate" or "myo-inositol-hexaphosphate" refers to the molecule of the formula: 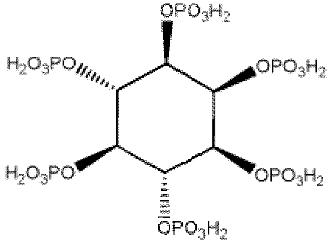 and the pharmaceutically acceptable salts thereof, which include but are not limited to sodium, potassium, calcium, magnesium, zinc, and calcium-magnesium salts. For the purposes of the present invention, the phytic acid and its pharmaceutically acceptable salts can be used in free form as pure substances, extracts of plant species that contain them, such as, for example, whole rice extracts, or vehicled in plant species that contain them, which can be germs or external parts of the grains or fruits of wheat, oats, soy, almond, locust bean, etc.

In the present invention, the term "B6 vitamers" or "vitamin B6" is understood to refer to all of the compounds represented in the following molecule, as well as the pharmaceutically acceptable ionic forms thereof. 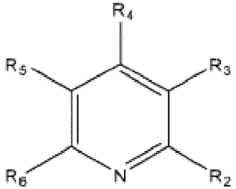
R₂, R₆= H, CH₃, (CH₂)ₙCH₃, OH, (CH₂)ₙOH, NH₂, (CH₂)ₙNH₂, N(CH₃)ₙ, (CH₂)ₙN(CH₃)ₙ.
R₃, R₅= H, NH₂, SH₂, OH, (CH₂)ₙNH₂, (CH₂)ₙSH₂, (CH₂)ₙCOH
R₄= NH₂, (CH₂)ₙNH₂, SH₂, (CH₂)ₙSH₂, COH, OH, (CH₂)ₙOH, (CH₂)ₙCOH, COOH, (CH₂)ₙCOOH

In another preferred embodiment, the medicament is in a form that is suitable for administration via oral, parenteral, intravenous, or enteral routes.

In another preferred embodiment, the composition of the invention comprises between 20-50% by weight of B6 vitamer or salts thereof and between 80-50% by weight of phytate or salts thereof. Non-limitative examples of the composition of the invention are as follows:

**Composition 1.**

| Compound | Quantity |
|---|---|
| Calcium-magnesium phytate | 300 mg |
| Vitamin B6 | 50 mg |

**Composition 2.**

| Compound | Quantity |
|---|---|
| Calcium-magnesium phytate | 350 mg |
| Vitamin B6 | 150 mg |

**Composition 3.**

| Compound | Quantity |
|---|---|
| Calcium-magnesium phytate | 400 mg |
| Vitamin B6 | 200 mg |

In another preferred embodiment, the composition is a pharmaceutical composition or a nutraceutical or functional food.

In the present invention, the term "nutraceutical" or "functional food" is understood to mean a food that has a beneficial effect on health. Likewise, the term nutraceutical can be applied to extracts or chemical compounds obtained from common foods. Examples of foods to which nutraceutical properties are attributed include olive oil, red wine, broccoli, soy, etc. The nutraceuticals are normally used in nutritional mixtures and in the pharmaceutical industry. Just as some foods can be classified as nutraceuticals, some nutritional supplements can, as well, such as fatty acids like omega-3 derivatives from fish oil and some plants or antioxidants and vitamins.

The combination of phytate or salts thereof and B6 vitamers or salts thereof can be administered in solid form (including granules, powder, or suppositories) or in liquid form (including solutions, suspensions, or emulsions). They can also be administered as they are or after having undergone operations such as sterilization, addition of preservatives, addition of stabilizers, or addition of emulsifying agents.

The B6 vitamers or pharmaceutically acceptable forms thereof can be used in their pure form or as part of extracts from plants containing them. Some examples include rice bran (4 mg/100g), pistachio powder (1.7 mg/100g), garlic powder (1.2 mg/100g), liver extract (1 mg/100g), bluefish (1 mg/100g), sunflower seeds (0.8 mg/100g), or hazelnuts (0.65 mg/100g).

The combination of phytate and vitamin B6 can be administered in solid form (including granules, powder, or suppositories) or in liquid form (including solutions, suspensions, or emulsions). They can also be administered as they are or after having undergone operations such as sterilization, addition of preservatives, addition of stabilizers, or addition of emulsifying agents.

Pharmaceutical compositions containing phytate and vitamin B6 will include a quantity of each active substance that enables the plasma levels of AGEs to be reduced effectively. In a preferred embodiment of the present invention, this reduction is also synergistic. The effective quantities for this purpose depend on factors such as the route of administration, the health of the individual, or the urinary oxalate levels, although these factors do not limit the inclusion of others that might help define the recommendable quantities. In any case, it will readily be understood that the quantities of each active substance that each individual will take will be determined by a specialist based on each individual circumstance.

Both the phytate and the vitamin B6, as well as combinations thereof, can be administered via oral, rectal, subcutaneous, intra-arterial, intramuscular, interspinal, intracranial, intravenous, or inhalation routes.

The conjoint administration of phytate or salts thereof and B6 vitamers or salts thereof can be combined with one or more compounds that facilitate the absorption thereof through the chosen route of administration. They can thus be administered with lactose, sucrose, talc, magnesium stearate, cellulose, calcium salts, gelatin, fatty acids, as well as with other similar substances.

The pharmaceutically acceptable adjuvants and vehicles that can be used in these compositions are the adjuvants and vehicles that are known by those skilled in the art and routinely used in the preparation of therapeutic compositions.

For use in therapy, the phytate or salts thereof and the B6 vitamers or salts thereof are preferably in a pharmaceutically acceptable or substantially pure form - that is, they have a pharmaceutically acceptable level of purity excluding the normal pharmaceutical additives such as diluents and carriers, and not including material that is considered toxic at normal dosing levels. The levels of purity for the active substance are preferably greater than 50%, more preferably greater than 70%, and even more preferably greater than 90%. In a preferred embodiment, the levels of the compound of formula (1) or salts or solvates thereof are greater than 95%.

Even though the therapeutic application of the present invention is aimed primarily at diabetic patients, phytate or a combination thereof with B6 vitamers may be applicable to any pathologic condition in which AGEs are produced. The presence of AGEs is associated with irreversible micro- and macrovascular complications which include neuropathy, nephropathy, atherosclerosis, vascular calcification, and neurodegenerative diseases (Alzheimer's and Parkinson's disease).

Throughout the description and the claims, the expression "comprises" and variants thereof are not intended to exclude other technical features, additives, components, or steps. For those skilled in the art, other objects, advantages, and features of the invention will be apparent in part from the description and in part from the practicing of the invention. The following examples and figures are provided for the sake of illustration and are not intended to limit the scope of the present invention.

### Brief Description of the Figures

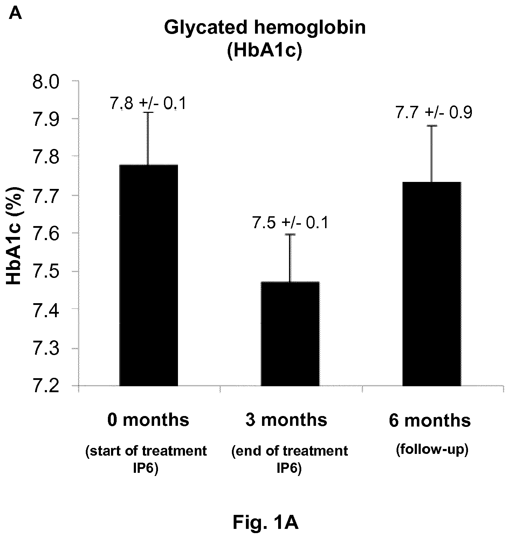

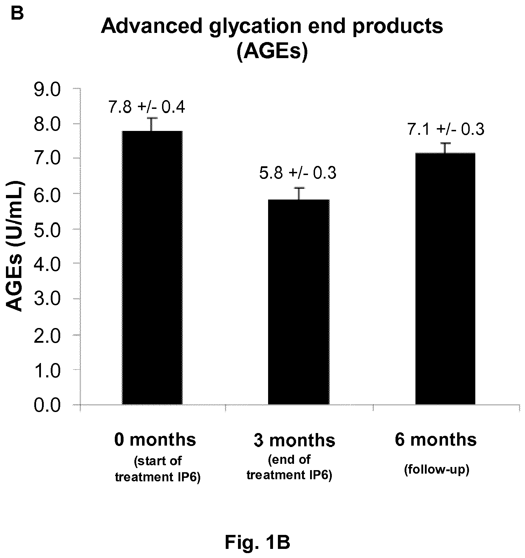

**Fig. 1** shows levels (average +/- SD) of glycated hemoglobin (A) and advanced glycation end-products (B) before and after treatment with phytate and at the follow-up visit. The differences between 0 and 3 months and 3 and 6 months were statistically significant (p<0.05).

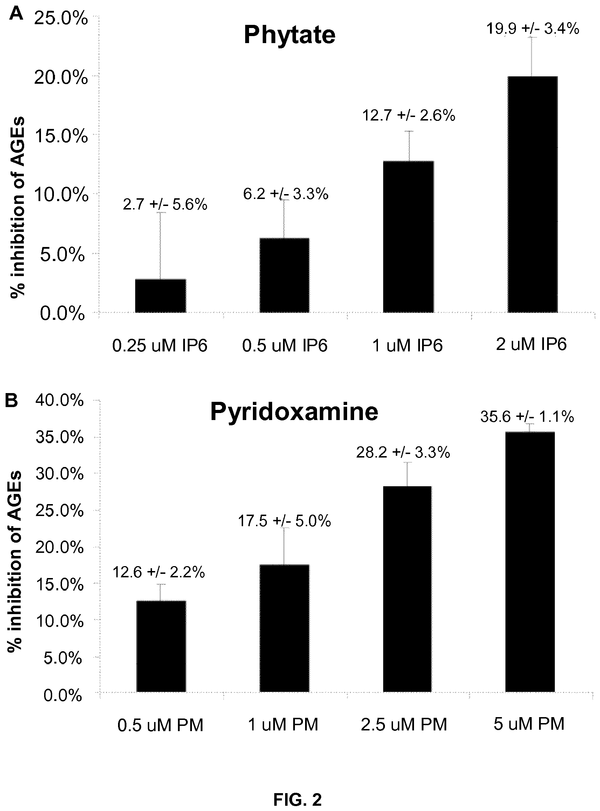

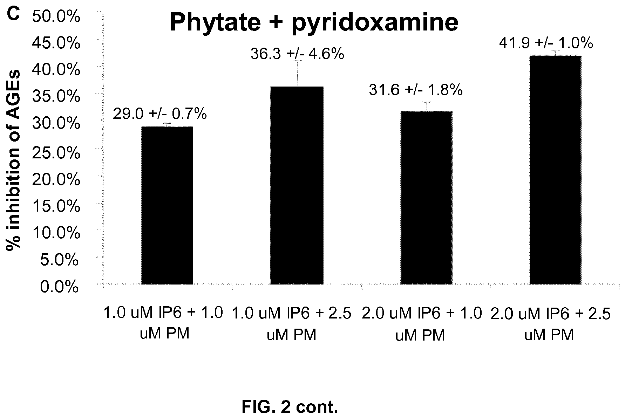

**Fig. 2** shows the percentage inhibition on the formation of AGEs of phytate (A), pyridoxamine (B), and phytate+pyridoxamine (C) with respect to the control test (without inhibitor). The percentage inhibition of phytate and pyridoxamine in combination was significantly greater (p<0.05) than that of these inhibitors separately with the same concentrations.

### Examples

The invention will be illustrated below by means of tests conducted by the inventors that demonstrate the effectiveness of the product of the invention.

### Example 1: Effect of the composition of the invention on diabetic patients.

A clinical trial was conducted in which 1,140 mg of phytate (in the form of phytin: calcium-magnesium salt) was administered daily to 33 diabetic patients for 3 months. Once this period had lapsed, the plasma levels of glycated hemoglobin (HbA1c) and AGEs were determined. A statistically significant decrease was observed both for glycated hemoglobin (on the order of 3.9%, p=0.017) and AGEs (on the order of 25.1%, p < 0.001) (see Fig. 1). After three months of consuming phytate, it was withdrawn, and the plasma levels of HbA1c and AGEs were determined again after three months without it; it was observed that patients' values increased again, returning to levels similar to the baselines observed prior to commencement of the study (see Fig. 1). An increase in the urinary excretion of phytate of 33.6% (p=0.026) was also observed. These findings show that phytate acts as an inhibitor of the formation of AGEs and protein glycation.

### Example 2: In vitro tests on the effect of phytate on the formation of AGEs

In an in vitro study, the inhibiting effect of phytate alone or in combination with pyridoxamine on the formation of AGEs was compared (see Fig. 2). It was observed that the effect of the combined use of phytate with pyridoxamine is superior to the effect of the two inhibitors separately in their physiological concentrations (phytate: 1 and 2 µM; pyridoxamine: 1 and 2.5 µM). In these studies, the formation of AGEs was induced by incubating a reaction mixture containing ribose (200 mM) (taken as the model reducing sugar), 1 mM arginine, 2 mM lysine (as glycation targets), and 2 µM Fe³ (as a glycation catalyzer) with various concentrations of phytate and pyridoxamine alone or in combination at 37°C for 7 days (in a 0.2 M phosphate buffer). The quantity of AGEs present at 7 days was determined by fluorescence. As can be seen in Fig. 2, 1 µM and 2 µM of phytate inhibited the formation of AGEs by 12.7% and 19.9%, respectively. On the other hand, 1 µM and 2.5 µM pyridoxamine inhibited the formation of AGEs by 17.5% and 28.2%, respectively, compared to the control group (without inhibitors). However, the combined use of 1 µM phytate with 1 µM or 2.5 µM pyridoxamine resulted in a greater inhibiting effect of 29.0% and 36.3%, respectively. The combined use of 2 µM phytate with 1 µM or 2.5 µM pyridoxamine resulted in an inhibition of the formation of AGEs of 31.6% and 41.9%, respectively. These results demonstrate that the inhibiting effect both of pyridoxamine and of phytate is greater in combination than when they are used separately, which reveals a clear complementary effect between the two.

These models show that a composition that comprises phytate in a form that also contains vitamin B6 can be used for the manufacture of medicaments or dietary supplements intended for the treatment of those pathologies that appear due to an accumulation of AGEs in the organism.

## Claims

1. A use of a composition that comprises phytic acid or any of the pharmaceutically acceptable salts thereof for the manufacture of a medicament for preventing the formation of glycation end-products in diabetic patients, with the medicament being prepared so as to be administered via oral, rectal, subcutaneous, intra-arterial, intramuscular, interspinal, intracranial, intravenous, or inhalation routes.

2. The use as set forth in claim 1, wherein the composition further comprises a B6 vitamer or any of the salts thereof.

3. The use as set forth in claim 2, wherein the B6 vitamer is selected from among pyridoxine, pyridoxal, or pyridoxamine.

4. The use as set forth in the preceding claim, wherein the vitamin B6 derivative is pyridoxamine.

5. The use as set forth in any one of the preceding claims, wherein the pharmaceutically acceptable salts of phytic acid are selected from among sodium phytate, potassium phytate, calcium phytate, magnesium phytate, zinc phytate, or combinations thereof.

6. The use as set forth in the preceding claim, wherein the phytic acid salt is calcium-magnesium phytate.

7. The use as set forth in any one of the preceding claims, wherein the phytic acid or salts thereof originate from a plant species that is rich in phytic acid or salts thereof, or from a vegetable extract from that species.

8. A use of a composition that comprises phytic acid or any of the pharmaceutically acceptable salts thereof, combined sequentially, simultaneously, or separately with a B6 vitamer, for the manufacture of a medicament for preventing the formation of glycation end-products in diabetic patients, with the medicament being prepared so as to be administered via oral, rectal, subcutaneous, intra-arterial, intramuscular, interspinal, intracranial, intravenous, or inhalation routes.

9. A composition that comprises phytic acid or any of the salts thereof and at least one derivative of vitamin B6 or any of the salts thereof, with the composition being prepared so as to be administered via oral, rectal, subcutaneous, intra-arterial, intramuscular, interspinal, intracranial, intravenous, or inhalation routes.

10. The composition as set forth in claim 9, wherein the B6 vitamer is selected from among pyridoxine, pyridoxal, or pyridoxamine.

11. The composition as set forth in the preceding claim, wherein the B6 vitamer is pyridoxamine.

12. The composition as set forth in any one of claims 9 to 11, wherein the phytic acid salt is selected from among sodium phytate, potassium phytate, calcium phytate, magnesium phytate, zinc phytate, or combinations thereof.

13. The composition as set forth in the preceding claim, wherein the phytic acid salt is calcium-magnesium phytate.

14. The composition as set forth in any one of claims 9 to 13, wherein the phytic acid or salts thereof originate from a plant species that is rich in phytic acid or salts thereof, or from a vegetable extract from that species.

15. The composition as set forth in any one of claims 9 to 14, wherein the composition is a pharmaceutical composition or a nutraceutical or functional food.

16. The use of the composition as set forth in any one of claims 9 to 15 for the manufacture of a medicament.
